# EUROPEAN PATENT SPECIFICATION

(11) **EP 1 140 243 B1**
(45) Date of publication and mention of the grant of the patent: **09.04.2003**
(21) Application number: 99962015.6
(22) Date of filing: 30.12.1999
(51) Int. Cl.: A61L 31/10, A61L 31/16, A61F 2/06

(54) **STENT GRAFTS WITH BIOACTIVE COATINGS**
STENT-TRANSPLANTATE MIT BIOAKTIVEN BESCHICHTUNGEN
PROTHESES ENDOVASCULAIRES A REVETEMENTS BIOACTIFS

(30) Priority: 31.12.1998 US 114731 P; 20.01.1999 US 116726 P
(43) Date of publication of application: 10.10.2001
(73) Proprietor: Angiotech Pharmaceuticals, Inc., Vancouver, British Columbia V6T 1Z4 (CA); UNIVERSITY OF BRITISH COLUMBIA, Vancouver, British Columbia V6T 1Z3 (CA)
(72) Inventor: MACHAN, Lindsay, S., Vancouver, British Columbia V6R 1A2 (CA); JACKSON, John, K., Vancouver, British Columbia V6N 2H9 (CA); HUNTER, William, L., Vancouver, British Columbia V5K 3L7 (CA)
(74) Representative: Gowshall, Jonathan Vallance
(86) International application number: CA9901237
(87) International publication number: WO00040278

(56) References cited:
- EP-A- 0 623 354
- EP-A- 0 734 721
- EP-A- 0 850 651
- WO-A-91/17789
- WO-A-93/06792
- WO-A-94/01483
- WO-A-94/13268
- WO-A-97/46268
- WO-A-97/46590
- US-A- 5 383 928
- US-A- 5 725 568

## Description

### TECHNICAL FIELD

The present invention relates generally to methods and devices, and more specifically, to methods for preparing stent grafts to make them more adherent to, or, more readily incorporated within a vessel wall.

### BACKGROUND OF THE INVENTION

Stent grafts have been developed in order to not only simply hold open a passageway, but also to bridge across diseased vasculature from healthy vessel to healthy vessel. The most common application of stent grafts is to bypass an abdominal aortic aneurysm (AAA). Briefly, a stent graft is inserted over a guide wire, from the femoral or iliac artery and deployed within the aneurysm, resulting in maintenance of blood flow from an aorta of acceptable (usually normal) caliber above to a portion of aorta or iliac artery(s) of acceptable (usually normal) caliber below the aneurysm. The aneurysm sac is thus excluded. Blood within this excluded sac thromboses and the aneurysm thus has no flow within it, presumably reducing the pressure and thus its tendency to burst.

Presently available stent grafts however have a number of problems. For example, current stent grafts are prone to persistent leakage around the area of the stent graft. Hence, pressure within the sac stays at or near arterial pressure and there is still a risk of rupture. There are 3 common types of perigraft leakage. The first type is direct leakage around the stent graft. This can be persistent from the time of insertion because of poor sealing between the stent graft and vessel wall, or can develop later because the seal is lost. In addition, this problem can develop due to changes in the position or orientation of the stent graft in relation to the aneurysm as the aneurysm grows, shrinks, elongates or shortens with time after treatment. The second type of perigraft leak can occur because there are side arteries extending out the treated segment of blood vessel. Once the aneurysm is excluded by the device, flow can reverse within these blood vessels and continue to fill the aneurysm sac around the stent graft. The third type of perigraft leak can occur because of disarticulation of the device (in the case of modular devices) or because of the development of holes within the graft material because continuous pulsation of the vessel causes the graft material to rub against a metallic stent tyne eventually causing graft failure. Disarticulation of the device can develop due to changes in shape of the aneurysm as it grows, shrinks, elongates or shortens with time after treatment.

Stent grafts are also limited in their application to only selected patients with aneurysms. For example, endovascular stents are an advance in the treatment of AAA as they offer the avoidance of standard therapy, which is a major operation with a significant morbidity, mortality, long hospital stays, and prolonged recovery time. However, endovascular technology is only applicable to certain patients with AAA because (a) lack of a suitable route of access via the blood vessels to the intended site of deployment which prevents insertion of the device and (b) anatomy.

More specifically, in order to exclude an aneurysm, the graft material needs to be of a certain strength and durability or it will tear. Typically, this implies a Dacron or PTFE graft material of conventional "surgical" thickness as thickness is one parameter to convey strength to the material. The thickness in the graft portion of the device results in the need for delivery devices which can be up to 32 French (10.67mm diameter). This almost always requires surgical exposure of the insertion site, usually a common femoral artery and limits the application of the technology as a larger delivery device is more difficult to manipulate through the iliac artery to the intended site of delivery. Even "low profile" devices which use thinner graft material still are of a sufficient size that a surgical exposure of the blood vessel through which the device is inserted is almost always required. If the iliac arteries or aorta are very tortuous, (frequently the case in AAA), or heavily calcified and diseased (another frequent association with AAA), this may be a contraindication to treatment or cause of failure of attempted treatment because of inability to advance a device to the site of deployment or potential for iliac artery rupture.

Furthermore, a stent graft typically bridges a diseased artery (usually an aneurysm) extending from a portion of artery of acceptable caliber above to acceptable caliber below. To achieve a long lasting seal the artery of acceptable caliber above ("proximal neck") should be at least 1.5 cm long without a major branch vessel arising from it, and the artery of acceptable caliber below ("distal neck") should be at least 1.0 cm long without a major branch vessel arising within that 1 cm. Shorter "necks" at either end of the diseased segment, necks which are sloping rather than cylindrical, or necks which are smaller than the aneurysm but still dilated in comparison to the normal diameter for a vessel in this location predispose to failure of sealing around the stent graft or delayed perigraft leaks.

One further difficulty with present stent grafts is that over time certain devices have a tendency to migrate distally within the abdominal aorta. Such migration results in device failure, perigraft leak and vessel occlusion.

Finally, there is long term uncertainty about the entire stent graft technology as a treatment for AAA. Standard open aneurysm repair is extremely durable. Uncertainties about endovascular stent grafts include whether they will lower the aneurysm rupture rate, rate of perigraft leak, device migration, ability to effectively exclude aneurysms over a long term, and device rupture or disarticulation.

The present invention discloses methods for preparing, and devices related to stent grafts, and further provides other related advantages.

### SUMMARY OF THE INVENTION

Briefly stated, the present invention provides stent grafts as well as methods for making these grafts. Within one aspect of the invention stent grafts are provided which induce adhesion or fibrosis in vessel walls, thus increasing or accelerating adherence of the stent graft to the vessel wall. Within various embodiments, such adhesion or fibrosis is induced by release of an agent from the stent graft.

Within related aspects of the present invention, stent grafts are provided comprising an endoluminal stent and a graft, wherein the stent graft releases an agent which induces the *in vivo* adhesion of the stent graft to vessel walls. As utilized herein, "induces adhesion to vessel walls" should be understood to refer to agents or compositions which increase or accelerates a reaction between the stent graft and the vessel wall, such that the position of the stent graft is fixed within the vessel. "Release of an agent" refers to any statistically significant presence of the agent, or a subcomponent thereof, which has disassociated from the stent graft.

Within a related aspect, stent grafts are provided comprising an endoluminal stent and a graft, wherein the stent graft induces or accelerates an *in vivo* fibrotic reaction causing the stent graft to adhere to vessel wall.

Within related aspects, stent grafts are constructed so that the graft itself is comprised of materials, which induce adhesion or fibrosis with vessel walls.

Within various embodiments of the invention, the stent graft is coated with a composition or compound, which delays the onset of adhesion or fibrosis. Representative examples of such agents include heparin, PLGA/MePEG, PLA, and polyethylene glycol. Within further embodiments the stent graft is activated prior to use (e.g., the agent is first activated from a previously inactive agent to an active agent, or, the stent graft is activated from a previously inactive stent graft to one that induces or accelerates an *in vivo* fibrotic reaction.). Such activation may be accomplished either before insertion, during insertion, or, subsequent to insertion.

Within one embodiment of the invention, the stent graft is adapted to release a vessel wall irritant. Representative examples of such irritants talcum powder, metallic beryllium, and silica. Other agents which may be released by the stent graft include components of extracellular matrix, fibronectin, polylysine, ethylenevinylacetate, and inflammatory cytokines such as TGFβ, PDGF, VEGF, bFGF, TNFα, NGF, GM-CSF, IGF-a, IL-1, IL-8, IL-6, and growth hormone, and adhesives such as cyanoacrylate.

A wide variety of stent grafts may be utilized within the context of the present invention, depending on the site and nature of treatment desired. Stent grafts may be, for example, bifurcated or tube grafts, cylindrical or tapered, self-expandable or balloon-expandable, unibody, or, modular. Moreover, the stent graft may be adapted to release the desired agent at only the distal ends, or along the entire body of the stent graft.

The stent grafts according to the present invention find utility in treating patients having aneurysms (*e.g.*, abdominal, thoracic, or iliac aortic aneurysms), for bypassing a diseased portion of a vessel, or for creating communication or a passageway between one vessel and another (*e.g*., artery to vein or vice versa, or artery to artery or vein to vein), such that risk of rupture of the aneurysm is reduced. As utilized herein, it should be understood that 'reduction in the risk of rupture' or 'prevention of the risk of rupture' refers to a statistically significant reduction in the, number, timing, or, rate of rupture, and not to a permanent prohibition of any rupture.

Within yet other aspects of the present invention methods are provided for manufacturing stent grafts, comprising the step of coating (*e.g.*, spraying, dipping, or, wrapping) a stent graft with an agent which induces adhesion of the stent graft to vessel walls (including for example, induction of an in vivo fibrotic reaction with vessel walls). Within related aspects, the stent graft can be constructed with materials, which release, or, by themselves induce adhesion or fibrosis with vessel walls.

These and other aspects of the present invention will become evident upon reference to the following detailed description and attached drawings. In addition, various references are set forth herein which describe in more detail certain procedures or compositions.

### BRIEF DESCRIPTION OF THE DRAWINGS

Figure 1 is a schematic illustration of one representative stent graft. Dashed lines indicate coating of the graft with a desired agent at each end of the graft.

Figure 2 is a cross-sectional view of the stent graft illustrated in Figure 1.

### DETAILED DESCRIPTION OF THE INVENTION

### Definitions

Prior to setting forth the invention, it may be helpful to an understanding thereof to first set forth definitions of certain terms that is used hereinafter.

"Stent graft" refers to devices comprising a graft or wrap (composed of a textile, polymer, or other suitable material such as biological tissue) which maintains the flow of fluids (*e.g*., blood) from one portion of a vessel to another, and an endovascular scaffolding or stent which holds open a body passageway and/or supports the graft or wrap. The graft or wrap may be woven within a stent, contained within the lumen of a stent and/or exterior to a stent.

As discussed above, the present invention provides methods and devices relating to stent grafts, which greatly increase the success and application of stent grafts. In particular, such stent grafts can accelerate or enhance occlusion of the lumen of the blood vessel outside the stent graft, and increase the strength and durability of the graft portion of the device.

Described in more detail below are methods for constructing stent grafts, and methods for generating stent grafts which adhere to a vessel wall, and methods for utilizing such stent grafts.

### Construction of Stent Grafts

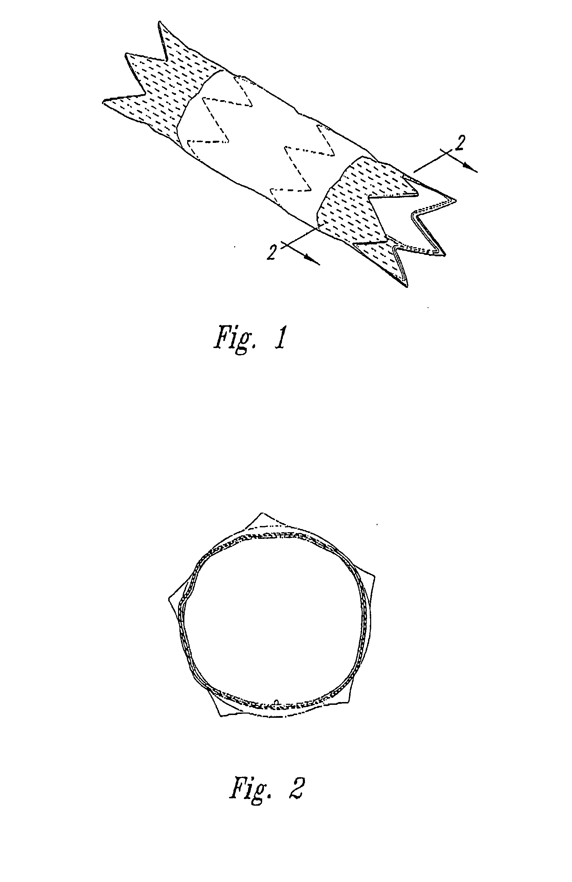

As noted above, stent grafts refers to devices comprising a graft or wrap which maintains the flow of fluids (*e.g*., blood) from one portion of a vessel to another, or from one blood vessel to another, and an endovascular scaffolding or stent which holds open a body passageway and/or supports the graft or wrap. One representative stent graft is illustrated in Figures 1 and 2.

The graft portion of the stent may be composed of a textile, polymer, or other suitable material such as biological tissue. Representative examples of suitable graft materials include textiles such as nylon, Orlon, Dacron, or woven Teflon, and non-textiles such as expanded polytetrafluroethylene (PTFE). The graft or wrap may be woven within a stent, contained within the lumen of a stent and/or exterior to a stent.

The endovascular scaffolding or stent is adapted to hold open a body passageway and/or support the graft or wrap. Representative examples of stent grafts, and methods for making and utilizing such grafts are described in more detail in U.S. Patent No. 5,810,870 entitled "Intraluminal Stent Graft"; U.S. Patent No. 5,776,180 entitled "Bifurcated Endoluminal Prosthesis"; U.S. Patent No. 5,755,774 entitled "Bistable Luminal Graft Endoprosthesis"; U.S. Patent Nos. 5,735,892 and 5,700,285 entitled "Intraluminal Stent Graft"; U.S. Patent No. 5,723,004 entitled "Expandable Supportive Endoluminal Grafts"; U.S. Patent No. 5,718,973 entitled "Tubular Intraluminal Graft"; U.S. Patent No. 5,716,365 entitled "Bifurcated Endoluminal Prosthesis"; U.S. Patent No. 5,713,917 entitled "Apparatus and Method for Engrafting a Blood Vessel"; U.S. Patent No. 5,693,087 entitled "Method for Repairing an Abdominal Aortic Aneurysm"; U.S. Patent No. 5,683,452 entitled "Method for Repairing an Abdominal Aortic Aneurysm"; U.S. Patent No. 5,683,448 entitled "Intraluminal Stent and Graft"; U.S. Patent No. 5,653,747 entitled "Luminal Graft Endoprosthesis and Manufacture Thereof"; U.S. Patent No. 5,643,208 entitled "Balloon Device of Use in Repairing an Abdominal Aortic Aneurysm"; U.S. Patent No. 5,639,278 entitled "Expandable Supportive Bifurcated Endoluminal Grafts"; U.S. Patent No. 5,632,772 entitled "Expandable Supportive Branched Endoluminal Grafts"; U.S. Patent No. 5,628,788 entitled "Self-Expanding Endoluminal Stent-Graft"; U.S. Patent No. 5,591,229 entitled "Aortic Graft for Repairing an Abdominal Aortic Aneurysm"; U.S. Patent No. 5,591,195 entitled "Apparatus and Methods for Engrafting a Blood Vessel"; U.S. Patent No. 5,578,072 entitled "Aortic Graft and Apparatus for Repairing an Abdominal Aortic Aneurysm"; U.S. Patent No. 5,578,071 entitled "Aortic Graft"; U.S. Patent No. 5,571,173 entitled "Graft to Repair a Body Passageway"; U.S. Patent No. 5,571,171 entitled "Method for Repairing an Artery in a Body"; U.S. Patent No. 5,522,880 entitled "Method for Repairing an Abdominal Aortic Aneurysm"; U.S. Patent No. 5,405,377 entitled "Intraluminal Stent"; and U.S. Patent No. 5,360,443 entitled "Aortic Graft for Repairing an Abdominal Aortic Aneurysm".

### Compositions and Methods for Generating Stent Grafts which Adhere to a Vessel Wall

Stent grafts of the present invention are coated with, or otherwise adapted to release an agent which induces adhesion to vessel walls. Stent grafts may be adapted to release such an agent by (a) directly affixing to the implant or device a desired agent or composition (*e.g*., by either spraying the stent graft with a polymer/drug film, or by dipping the implant or device into a polymer/drug solution, or by other covalent or noncovalent means); (b) by coating the stent graft with a substance such as a hydrogel which will in turn absorb the desired agent or composition; (c) by interweaving agent or composition coated thread into the stent graft (*e.g*., a polymer which releases the agent formed into a thread) into the implant or device; (d) by inserting a sleeve or mesh which is comprised of or coated with the desired agent or composition; (e) constructing the stent graft itself with the desired agent or composition; or (f) otherwise impregnating the stent graft with the desired agent or composition. Suitable adhesion inducing agents may be readily determined based upon the animal models provided in Example 14 (Screening Procedure for Assessment of Perigraft Reaction) and Example 15 (Animal Abdominal Aortic Aneurysm Model).

Representative examples of adhesion inducing agents include irritants (*e.g*., talcum powder, metallic beryllium and silica), components of extracellular matrix *(e.g.,* fibronectin); polymers (*e.g*., polylysine and ethylenevinylacetate); inflammatory cytokines *(e.g.,* TGFβ, PDGF, VEGF, bFGF, TNFα, NGF, GM-CSF, IGF-a, IL-1, IL-8, IL-6, and growth hormone); and inflammatory microcrystals (*e.g.*, crystalline minerals such as crystalline silicates). Other representative examples include Monocyte chemotactic protein, fibroblast stimulating factor 1, histamine, fibrin or fibrinogen, endothelin-1, angiotensin II, bovine collagen, bromocriptine, methylsergide, methotrexate, N-carboxybutyl chitosan, carbon tetrachloride, Thioacetamide, talcum powder, Metallic beryllium (or its oxides), Quartz dust, Polylysine, Fibrosin, and ethanol.

Optionally, within one embodiment of the invention a desired adhesion-inducing agent may be admixed with, blended with, conjugated to, or, otherwise modified to contain as a composition a polymer, which may be either biodegradable or non-biodegradable. Representative examples of biodegradable compositions include albumin, collagen, gelatin, hyaluronic acid, starch, cellulose (methylcellulose, hydroxypropylcellulose, hydroxypropylmethylcellulose, carboxymethylcellulose, cellulose acetate phthalate, cellulose acetate succinate, hydroxypropylmethylcellulose phthalate), casein, dextrans, polysaccharides, fibrinogen, poly(D,L-lactide), poly(D,L-lactide-co-glycolide), poly(glycolide), poly(hydroxybutyrate), poly(alkylcarbonate) and poly(orthoesters), polyesters, poly(hydroxyvaleric acid), polydioxanone, poly(ethylene terephthalate), poly(malic acid), poly(tartronic acid), polyanhydrides, polyphosphazenes, poly(amino acids) and their copolymers (*see generally*, Illum, L., Davids, S.S. (eds.) "Polymers in Controlled Drug Delivery" Wright, Bristol, 1987; Arshady, *J. Controlled Release 17*:1-22, 1991; Pitt, *Int. J. Phar. 59*:173-196, 1990; Holland et al., *J. Controlled Release 4*:155-0180, 1986). Representative examples of non-degradable polymers include poly(ethylene-vinyl acetate) ("EVA") copolymers, silicone rubber, acrylic polymers (polyacrylic acid, polymethylacrylic acid, polymethylmethacrylate, polyalkylcynoacrylate), polyethylene, polypropylene, polyamides (nylon 6,6), polyurethane, poly(ester urethanes), poly(ether urethanes), poly(ester-urea), polyethers (poly(ethylene oxide), poly(propylene oxide), Pluronics and poly(tetramethylene glycol)), silicone rubbers and vinyl polymers (polyvinylpyrrolidone, poly(vinyl alcohol), poly(vinyl acetate phthalate). Polymers may also be developed which are either anionic (*e.g*., alginate, carrageenan, carboxymethyl cellulose and poly(acrylic acid), or cationic (*e.g*., chitosan, poly-L-lysine, polyethylenimine, and poly(allyl amine)) *(see generally,* Dunn et al., *J. Applied Polymer Sci. 50*:353-365, 1993; Cascone et al., *J. Materials Sci.: Materials in Medicine 5*:770-774, 1994; Shiraishi et al., *Biol. Pharm. Bull. 16*(11):1164-1168, 1993; Thacharodi and Rao, *Int'l J*. *Pharm. 120*:115-118, 1995; Miyazaki et al., *Int'l J. Pharm. 118*:257-263, 1995). Particularly preferred polymeric carriers include poly(ethylene-vinyl acetate), polyurethanes, poly (D,L-lactic acid) oligomers and polymers, poly (L-lactic acid) oligomers and polymers, poly (glycolic acid), copolymers of lactic acid and glycolic acid, poly (caprolactone), poly (valerolactone), polyanhydrides, copolymers of poly (caprolactone) or poly (lactic acid) with a polyethylene glycol (*e.g*., MePEG), and blends, admixtures, or co-polymers of any of the above. Other preferred polymers include polysaccharides such as hyaluronic acid, chitosan and fucans, and copolymers of polysaccharides with degradable polymers.

All of the above polymers may be blended or copolymerized in various compositions as required.

Polymeric carriers can be fashioned in a variety of forms, with desired release characteristics and/or with specific desired properties. For example, polymeric carriers may be fashioned to release a therapeutic agent upon exposure to a specific triggering event such as pH (*see, e.g.,* Heller et al., "Chemically Self-Regulated Drug Delivery Systems," in *Polymers in Medicine III*, Elsevier Science Publishers B.V., Amsterdam, 1988, pp. 175-188; Kang et al., *J. Applied Polymer Sci. 48*:343-354, 1993; Dong et al., *J. Controlled Release 19:171-178, 1992;* Dong and Hoffman, *J. Controlled Release 15*:141-152, 1991; Kim et al., *J. Controlled Release 28*:143-152, 1994; Cornejo-Bravo et al., *J. Controlled Release 33*:223-229, 1995; Wu and Lee, *Pharm. Res. 10*(10):1544-1547, 1993; Serres et al., *Pharm. Res. 13*(2):196-201, 1996; Peppas, "Fundamentals of pH- and Temperature-Sensitive Delivery Systems," in Gurny et al. (eds.), *Pulsatile Drug Delivery,* Wissenschaftliche Verlagsgesellschaft mbH, Stuttgart, 1993, pp. 41-55; Doelker, "Cellulose Derivatives," 1993, in Peppas and Langer (eds.), *Biopolymers I,* Springer-Verlag, Berlin). Representative examples of pH-sensitive polymers include poly(acrylic acid) and its derivatives (including for example, homopolymers such as poly(aminocarboxylic acid); poly(acrylic acid); poly(methyl acrylic acid), copolymers of such homopolymers, and copolymers of poly(acrylic acid) and acrylmonomers such as those discussed above. Other pH sensitive polymers include polysaccharides such as cellulose acetate phthalate; hydroxypropylmethylcellulose phthalate; hydroxypropylmethylcellulose acetate succinate; cellulose acetate trimellilate; and chitosan. Yet other pH sensitive polymers include any mixture of a pH sensitive polymer and a water-soluble polymer.

Likewise, polymeric carriers can be fashioned which are temperature sensitive (*see, e.g.*, Chen et al., "Novel Hydrogels of a Temperature-Sensitive Pluronic Grafted to a Bioadhesive Polyacrylic Acid Backbone for Vaginal Drug Delivery," in *Proceed. Intern. Symp. Control. Rel. Bioact. Mater. 22*:167-168, Controlled Release Society, Inc., 1995; Okano, "Molecular Design of Stimuli-Responsive Hydrogels for Temporal Controlled Drug Delivery," in *Proceed. Intern. Symp. Control. Rel. Bioact. Mater. 22*:111-112, Controlled Release Society, Inc., 1995; Johnston et al., *Pharm. Res. 9*(3):425-433, 1992; Tung, *Int'l J. Pharm. 107*:85-90, 1994; Harsh and Gehrke, *J. Controlled Release 17*:175-186, 1991; Bae et al., *Pharm. Res. 8*(4):531-537, 1991; Dinarvand and D'Emanuele, *J. Controlled Release 36*:221-227, 1995; Yu and Grainger, "Novel Thermo-sensitive Amphiphilic Gels: Poly N-isopropylacrylamide-co-sodium acrylate-co-n-N-alkylacrylamide Network Synthesis and Physicochemical Characterization," Dept. of Chemical & Biological Sci., Oregon Graduate Institute of Science & Technology, Beaverton, OR, pp. 820-821; Zhou and Smid, "Physical Hydrogels of Associative Star Polymers," Polymer Research Institute, Dept. of Chemistry, College of Environmental Science and Forestry, State Univ. of New York, Syracuse, NY, pp. 822-823; Hoffman et al., "Characterizing Pore Sizes and Water 'Structure' in Stimuli-Responsive Hydrogels," Center for Bioengineering, Univ. of Washington, Seattle, WA, p. 828; Yu and Grainger, "Thermo-sensitive Swelling Behavior in Crosslinked N-isopropylacrylamide Networks: Cationic, Anionic and Ampholytic Hydrogels," Dept. of Chemical & Biological Sci., Oregon Graduate Institute of Science & Technology, Beaverton, OR, pp. 829-830; Kim et al., *Pharm. Res. 9*(3):283-290, 1992; Bae et al., *Pharm. Res. 8*(5):624-628, 1991; Kono et al., *J. Controlled Release 30*:69-75, 1994; Yoshida et al., *J. Controlled Release 32*:97-102, 1994; Okano et al., *J. Controlled Release 36*:125-133, 1995; Chun and Kim, *J*. *Controlled Release 38*:39-47, 1996; D'Emanuele and Dinarvand, *Int'l J. Pharm. 118*:237-242, 1995; Katono et al., *J. Controlled Release 16*:215-228, 1991; Hoffman, "Thermally Reversible Hydrogels Containing Biologically Active Species," in Migliaresi et al. (eds.), *Polymers in Medicine III*, Elsevier Science Publishers B.V., Amsterdam, 1988, pp. 161-167; Hoffman, "Applications of Thermally Reversible Polymers and Hydrogels in Therapeutics and Diagnostics," in *Third International Symposium on Recent Advances in Drug Delivery Systems*, Salt Lake City, UT, Feb. 24-27, 1987, pp. 297-305; Gutowska et al., *J. Controlled Release 22*:95-104, 1992; Palasis and Gehrke, *J. Controlled Release 18*:1-12, 1992; Paavola et al., *Pharm. Res. 12*(12):1997-2002, 1995).

Representative examples of thermogelling polymers, and their gelatin temperature (LCST (°C)) include homopolymers such as poly(N-methyl-N-n-propylacrylamide), 19.8; poly(N-n-propylacrylamide), 21.5; poly(N-methyl-N-isopropylacrylamide), 22.3; poly(N-n-propylmethacrylamide), 28.0; poly(N-isopropylacrylamide), 30.9; poly(N, n-diethylacrylamide), 32.0; poly(N-isopropylmethacrylamide), 44.0; poly(N-cyclopropylacrylamide), 45.5; poly(N-ethylmethyacrylamide), 50.0; poly(N-methyl-N-ethylacrylamide), 56.0; poly(N-cyclopropylmethacrylamide), 59.0; poly(N-ethylacrylamide), 72.0. Moreover thermogelling polymers may be made by preparing copolymers between (among) monomers of the above, or by combining such homopolymers with other water-soluble polymers such as acrylmonomers (*e.g*., acrylic acid and derivatives thereof such as methylacrylic acid, acrylate and derivatives thereof such as butyl methacrylate, acrylamide, and N-n-butyl acrylamide).

Other representative examples of thermogelling polymers include cellulose ether derivatives such as hydroxypropyl cellulose, 41°C; methyl cellulose, 55°C; hydroxypropylmethyl cellulose, 66°C; and ethylhydroxyethyl cellulose, and Pluronics such as F-127, 10 - 15°C; L-122, 19°C; L-92, 26°C; L-81, 20°C; and L-61, 24°C.

Therapeutic agents may be linked by occlusion in the matrices of the polymer, bound by covalent linkages, or encapsulated in microcapsules. Within certain preferred embodiments of the invention, therapeutic compositions are provided in non-capsular formulations such as microspheres (ranging from nanometers to micrometers in size), pastes, threads of various size, films and sprays.

Within certain aspects of the present invention, the therapeutic composition should be biocompatible, and release one or more therapeutic agents over a period of several hours, days, or, months. For example, "quick release" or "burst" therapeutic compositions are provided that release greater than 10%, 20%, or 25% (w/v) of a therapeutic agent over a period of 7 to 10 days. Such "quick release" compositions should, within certain embodiments, be capable of releasing chemotherapeutic levels (where applicable) of a desired agent. Within other embodiments, "slow release" therapeutic compositions are provided that release less than 1% (w/v) of a therapeutic agent over a period of 7 to 10 days. Further, therapeutic compositions of the present invention should preferably be stable for several months and capable of being produced and maintained under sterile conditions.

Within certain aspects of the present invention, therapeutic compositions may be fashioned in any size ranging from 50 nm to 500 µm, depending upon the particular use. Alternatively, such compositions may also be readily applied as a "spray", which solidifies into a film or coating. Such sprays may be prepared from microspheres of a wide array of sizes, including for example, from 0.1 µm to 3 µm, from 10 µm to 30 µm, and from 30 µm to 100 µm.

Therapeutic compositions of the present invention may also be prepared in a variety of "paste" or gel forms. For example, within one embodiment of the invention, therapeutic compositions are provided which are liquid at one temperature (*e.g*., temperature greater than 37°C, such as 40°C, 45°C, 50°C, 55°C or 60°C), and solid or semi-solid at another temperature (*e.g.*, ambient body temperature, or any temperature lower than 37°C). Such "thermopastes" may be readily made utilizing a variety of techniques (see, *e.g.,* PCT Publication WO 98/24427). Other pastes may be applied as a liquid which solidify *in vivo* due to dissolution of a water-soluble component of the paste, and precipitation of encapsulated drug into the aqueous body environment.

Within yet other aspects of the invention, the therapeutic compositions of the present invention may be formed as a film. Preferably, such films are generally less than 5, 4, 3, 2, or 1 mm thick, more preferably less than 0.75 mm, 0.5 mm, 0.25 mm, or, 0.10 mm thick. Films can also be generated of thicknesses less than 50 µm, 25 µm or 10 µm. Such films are preferably flexible with a good tensile strength (*e.g.*, greater than 50, preferably greater than 100, and more preferably greater than 150 or 200 N/cm²), good adhesive properties *(i.e.,* adheres to moist or wet surfaces), and have controlled permeability.

Within certain embodiments of the invention, the therapeutic compositions may also comprise additional ingredients such as surfactants (*e.g.*, Pluronics such as F-127, L-122, L-101, L-92, L-81, and L-61).

Within further aspects of the present invention, polymeric carriers are provided which are adapted to contain and release a hydrophobic compound, the carrier containing the hydrophobic compound in combination with a carbohydrate, protein or polypeptide. Within certain embodiments, the polymeric carrier contains or comprises regions, pockets, or granules of one or more hydrophobic compounds. For example, within one embodiment of the invention, hydrophobic compounds may be incorporated within a matrix which contains the hydrophobic compound, followed by incorporation of the matrix within the polymeric carrier. A variety of matrices can be utilized in this regard, including for example, carbohydrates and polysaccharides such as starch, cellulose, dextran, methylcellulose, chitosan and hyaluronic acid, proteins or polypeptides such as albumin, collagen and gelatin. Within alternative embodiments, hydrophobic compounds may be contained within a hydrophobic core, and this core contained within a hydrophilic shell.

Other carriers that may likewise be utilized to contain and deliver the therapeutic agents described herein include: hydroxypropyl β cyclodextrin (Cserhati and Hollo, *Int. J. Pharm. 108*:69-75, 1994), liposomes *(see, e.g*., Sharma et al., *Cancer Res. 53*:5877-5881, 1993; Sharma and Straubinger, *Pharm. Res. 11*(60):889-896, 1994; WO 93/18751; U.S. Patent No. 5,242,073), liposome/gel (WO 94/26254), nanocapsules (Bartoli et al., *J. Microencapsulation 7*(2):191-197, 1990), micelles (Alkan-Onyuksel et al., *Pharm. Res. 11*(2):206-212, 1994), implants (Jampel et al., *Invest. Ophthalm. Vis. Science 34*(11):3076-3083, 1993; Walter et al., *Cancer Res. 54*:22017-2212, 1994), nanoparticles (Violante and Lanzafame PAACR), nanoparticles - modified (U.S. Patent No. 5,145,684), nanoparticles (surface modified) (U.S. Patent No. 5,399,363), taxol emulsion/solution (U.S. Patent No. 5,407,683), micelle (surfactant) (U.S. Patent No. 5,403,858), synthetic phospholipid compounds (U.S. Patent No. 4,534,899), gas borne dispersion (U.S. Patent No. 5,301,664), liquid emulsions, foam, spray, gel, lotion, cream, ointment, dispersed vesicles, particles or droplets solid- or liquid- aerosols, microemulsions (U.S. Patent No. 5,330,756), polymeric shell (nano- and microcapsule) (U.S. Patent No. 5,439,686), taxoid-based compositions in a surface-active agent (U.S. Patent No. 5,438,072), emulsion (Tarr et al., *Pharm Res. 4*: 62-165, 1987), nanospheres (Hagan et al., *Proc. Intern. Symp. Control Rel. Bioact. Mater. 22,* 1995; Kwon et al., *Pharm Res. 12*(2):192-195; Kwon et al., *Pharm Res. 10*(7):970-974; Yokoyama et al., *J. Contr. Rel. 32*:269-277, 1994; Gref et al., *Science 263*:1600-1603, 1994; Bazile et al., *J. Pharm. Sci. 84*:493-498, 1994) and implants (U.S. Patent No. 4,882,168).

Within further aspects of the invention, the stent graft which induces *in vivo* adhesion and/or an *in vivo* fibrotic reaction with vessel walls is further coated with a compound or compositions which delays the release of and/or activity of the adhesion causing or fibrosis inducing agent. Representative examples of such agents include biologically inert materials such as gelatin, PLGA/MePEG film, PLA, or polyethylene glycol, as well as biologically active materials such as heparin (e.g., to induce coagulation).

For example, in one embodiment of the invention the active agent of the stent graft (e.g., poly-1-lysine, fibronectin, or chitosan) is coated with a physical barrier. Such barriers can include inert biodegradable materials such as gelatin, PLGA/MePEG film, PLA, or polyethylene glycol among others. In the case of PLGA/ MePEG, once the PLGA/ MePEG becomes exposed to blood, the MePEG will dissolve out of the PLGA, leaving channels through the PLGA to underlying layer of biologically active substance (e.g. poly-1-lysine, fibronectin, or chitosan) which then can initiate its biological activity.

Protection of a biologically active surface can also be accomplished by coating the surface with an inert molecule that prevents access to the active site through steric hindrance, or by coating the surface with an inactive form of the biologically active substance, which is later activated. For example, the stent graft can be coated with an enzyme which causes either release of the biologically active agent, or activates the biologically active agent, or cleaves a non-active coating to expose the active agent.

For example, within one embodiment a stent graft is coated with a biologically active substance, such as poly-1-lysine in the usual manner. The stent graft is then further coated with a polymer (such as polyethylene glycol methyl ether, amino terminated to bind some of the active sites on the poly-1-lysine molecule using a cleavable cross-linking agent such as, for example, dithiobis(succinimidyl propionate) or any other similar agent, e.g., dtbp, dtme, dtssp, (available from Pierce, Rockford, Illinois, USA) which creates a protective agent around the active sites. The stent graft may then be further coated with a mixture of dithiothreitol, B- mercaptothanol, sodium borohydride (examples of S - S cleaving agents) in a slow release polymer. Once the stent graft is fully deployed, excluding the aneurysm, the slow release polymer will release the cleaving agent, remove the protective polymer and expose the active agent.

Another example of a suitable surface coating is heparin which can be coated on top of the biologically active agent (e.g. poly 1 lysine, fibronectin, or chitosan). The presence of heparin delays coagulation. As the heparin or other anticoagulant dissolved away, the anticoagulant activity would stop, and the newly exposed biologically active agent (e.g. poly 1 lysine, fibronectin, or chitosan) could initiate its intended action.

In another strategy, the stent graft can be coated with an inactive form of the biologically active coating, which is then activated once the stent graft is deployed. Such activation could be achieved by injecting another material into the aneurysm sac after the stent graft is deployed. In this iteration, the graft material could be coated with an inactive form of the biologically active substance, such as poly 1 lysine, fibronectin, or chitosan, applied in the usual manner. Prior to the deployment of the aortic segment of the device, a catheter would be placed within the aneurysm sac via the opposite iliac artery, via an upper limb vessel such as a brachial artery, or via the same vessel as the aortic segment is inserted through so that once the stent graft is deployed, this catheter will be inside the aneurysm sac, but outside the stent graft. The stent graft would then be deployed in the usual manner. Once the stent graft is fully deployed, excluding the aneurysm, the activating substance is injected into the aneurysm sac around the outside of the stent graft.

One example of this method would be coating the graft material with the biologically active substance, such as poly-1-lysine, fibronectin, or chitosan, in the usual manner. The biologically active coating would then be covered with polyethylene glycol and these 2 substances would then be bonded through an ester bond using a condensation reaction. Prior to the deployment of the aortic segment of the device, a catheter would be placed within the aneurysm sac via the opposite iliac artery, via an upper limb vessel such as a brachial artery, or via the same vessel as the aortic segment is inserted through. Once the stent graft is fully deployed, excluding the aneurysm, an esterase is injected into the aneurysm sac around the outside of the stent graft, which will cleave the bond between the ester and the biologically active substance, allowing the substance to initiate the desired reaction.

In further embodiments, it may be desirable to induce a blood vessel wall reaction or adhesion at each end of the stent graft, but in the central portion induce another reaction, e.g., a "filler effect" to tighten the seal between the stent graft and the blood vessel wall, thus filling the excluded aneurysm, or coagulating blood within the aneurysm sac. This might be done by placing these substances along the entire length of the device, or by coating the ends of the device with an adhesive / fibrosis inducing agent, and the center portion with a combination of that agent, and a space occupying agent such as, for example "Water Lock" (G - 400, Grain Processing Corporation, Muscatine, IA). The space occupying agent can then be covered with a layer of PLGA/MePEG. Once the PLGA/ MePEG becomes exposed to blood, the MePEG will dissolve out of the PLGA, leaving channels through the PLGA to underlying layer of swelling material, which then swell considerably, impinging upon the lumen of the aneurysm. Other materials which might be used include hyaluronic acid, chitosan particles in nonaqueous media such as propylene glycol.

### Methods for Utilizing Stent Grafts

Stent grafts of the present invention may be utilized to induce a perigraft reaction or to otherwise create a tight adhesive bond between an endovascular prosthesis and the vascular wall. Such grafts provide a solution to the following common problems associated with endovascular stent graft technology.
1. *Persistent Perigraft Leaks -* a formation of fibrotic response or adhesion or tight adhesive bond between the proximal and distal interfaces between the stented portion of the stent graft and the vessel wall results in a more efficacious sealing around the device, and prevents late perigraft leaks arising at either end of the device even with a change in aneurysm morphology. Moreover, formation of a fibrous response or tight adhesion between the body of the graft and the aneurysm itself may result in occlusion of, or prevention of a perigraft leak due to retrograde flow (*i.e*., persistence of, or late reopening of the inferior mesenteric artery or lumbar arteries extending into the aneurysm). In addition, the coatings will induce or enhance blood clotting within the excluded aneurysm sac, which will further diminish the perigraft leak rate.
2. *Size of the Delivery Device* - one difficulty with present delivery devices is that they are quite large due to the required thickness of the stent graft. By inducing a reaction in the wall, which in itself conveys strength to the graft portion of the stent graft prosthesis, a thinner graft material might be utilized.
3. *Anatomic Factors which limit Patients with Aneurysmal Disease who are Candidates for Treatment with Endovascular Stent Grafts -* by inducing a fibrotic reaction or creating a tight durable adhesive bond between the prosthesis and the vascular wall at the proximal and distal margins of the grafted portion of the prosthesis, the length of the neck, particularly the proximal neck, can be shorter than the present suggested 1.5 centimeters as the fibrotic reaction or tight adhesion between graft and vessel wall will enhance sealing of the graft even when there is a short length of contact between the graft and vessel wall. (In an aneurysm, the walls are obviously dilated and thus extend away from the graft. When there is a long neck, apposition between graft material and vessel wall is only between the portion of vessel wall of "normal" diameter). In some cases, the portion of the vessel to which the device is to be anchored is dilated, *e.g*., a dilated iliac artery distal to an abdominal aortic aneurysm. If this segment of the vessel is too dilated, it tends to continue expansion after graft insertion, resulting in late perigraft leads. Patients with dilated iliac arteries or aortic neck might be denied therapy with uncoated devices. Creation of a firm bond between the graft and the vessel wall will prevent the neck from expanding further.
4. *Stent Graft Migration* - as the stent graft is firmly fixed against the vessel wall by more than just hooks or force of expansion between the stent graft and the vessel wall, migration of the stent graft or portions of the stent graft is prevented.
5. *Expansion of Applications of Stent Grafts -* Present applications of stent grafts for practical purposes are limited to situations where the stent graft is wholly deployed within a blood vessel. By strengthening the seal between the blood vessel wall and the device, this expands the possibility that the device can be used as an extravascular or even extra-anatomic conduit such as, but not limited to, between arteries, between an artery and a vein, or between veins, or between a vein and the peritoneal cavity. The expansion of stent grafts for these purposes is limited at least partially by the risk of leak of bodily fluid such as blood because of poor sealing at the site where the stent graft enters of leaves a body tube such as a blood vessel) or cavity.

Thus, stent grafts, which are adapted to adhere to vessel walls, can be utilized in a wide variety of therapeutic applications. For example, a stent graft can be utilized to connect one artery to another, either intra-anatomically (*e.g.*, to bypass aneurysms (*e.g*., carotid artery, thoracic aorta, abdominal aorta, subclavian artery, iliac artery, coronary artery, venous); to treat dissections (*e.g.*, carotid artery, coronary artery, iliac artery, subclavian artery); to bypass long segment disease (*e.g*., carotid artery, coronary artery, aorta, iliac artery, femoral artery, popliteal artery), or to treat local rupture (*e.g*., carotid artery, aorta, iliac artery, renal artery, femoral artery). Stent grafts might also be utilized extra-anatomically, for example, for arterial to arterial or arterial to venous dialysis fistula; or venous to venous for vascular access.

Stent grafts of the present invention may also be utilized to connect an artery to a vein (*e.g*., a dialysis fistula), or one vein to another *(e.g.,* a portacaval shunt, or venous bypass).

### A. Abdominal Aortic Aneurysms

In one representative example, stent grafts may be inserted into an Abdominal Aorta Aneurysm (AAA), in order to treat or prevent rupture of the abdominal aorta. Briefly, using sterile conditions, under appropriate anesthesia and analgesia, the common femoral artery is surgically exposed and an arteriotomy is performed after clamping of the artery. A guide wire is manipulated through the iliac arterial system and over this a catheter is inserted into the proximal abdominal aorta and an angiogram or intravascular ultrasound is performed. Subsequently the diagnostic catheter is exchanged over a guide wire for a delivery system, usually a sheath, containing the aortic portion of the stent graft system. If the device is an articulated bifurcated system, the most common iteration, than the ipsilateral iliac portion of the prosthesis is connected to the aortic portion. The device is deployed by releasing it from its constrained configuration in the case of a stent graft composed of self-expanding stents. If the stent graft skeleton is composed of balloon expandable stents, it is released by withdrawal of the sheath and inflating a balloon to expand the stent graft in place. After release of the aortic and ipsilateral iliac portion of the prosthesis, surgical exposure and cut down of the opposite iliac artery is performed and a guide wire is manipulated so that it passes through the deployed portion of the prosthesis. A similar delivery device containing the contralateral iliac limb of the prosthesis is then manipulated into the deployed aortic portion of the prosthesis and under fluoroscopic guidance is released in an appropriate position. The position is chosen so that the entire grafted portion of the stent graft sits below the renal arteries and preferably is deployed above the internal iliac arteries although one or both may be occluded. Depending on the patient's anatomy, further limb extensions may be inserted on either side. If the device is a tube graft, or a one piece bifurcated device, insertion via only one femoral artery may be required. A final angiogram is normally obtained by an angiographic catheter position with its distal portion in the upper abdominal aorta.

### B. Thoracic Aortic Aneurysm or Dissection

In another representative example, a stent graft may be utilized to treat or prevent a thoracic aortic aneurysm. Briefly, under appropriate anesthesia and analgesia, using sterile technique, a catheter is inserted via the right brachial artery into the ascending thoracic aorta and an angiogram performed. Once the proximal and distal boundaries of the diseased segment of the aorta to be treated are defined, an operative exposure of one of the common femoral arteries, usually the right, and an operative arteriotomy is performed. A guide wire is manipulated through the diseased segment of the aorta and over this, the delivery device, usually a sheath, is advanced so that the device is positioned across the diseased segment with the grafted portion of the stent immediately below the origin of the left subclavian artery. After contrast is injected to define the precise position of the stent graft, the device is deployed usually by withdrawing an outer sheath in the case of self-expanding stents so that the device is positioned immediately distal to the left subclavian artery and with its distal portion extending beyond the diseased portion of the thoracic aorta but above the celiac axis. A final angiogram is performed via the catheter inserted by the right brachial artery. The vascular access wounds are then closed.

### C. Delay of onset of activity of the stent coating

The time it takes to insert the device can be very long; for instance it theoretically could be hours between the time that the first part of a device (usually the aortic segment) is deployed and the second part of the device is deployed. It is not until all the parts of the device are inserted that an adequate exclusion of the aneurysm is achieved. In other words, the coating on the device may cause blood clots to form on or around the device. Because blood is rushing around as well as through the device until it is fully deployed, thereby excluding the aneurysm, such blood clots could be dislodged and washed downstream, or, might propagate distally. This could result in the inadvertent and undesirable occlusion or partial occlusion of blood vessels downstream from the intended site of insertion of the device, which the operator had intended to keep open. Several strategies may be employed to address such difficulties.

For example, as discussed in more detail above, stent grafts may be constructed which are designed to delay the onset of activity of the adhesion inducing, and/or fibrosis forming agent (e.g., by coating the stent graft with a material such as heparin or PLGA which delays adhesion or fibrosis). Alternatively, stent grafts may be constructed which are initially inert (i.e., do not substantially induce fibrosis or adhesion), and which are subsequently activated by another agent either at the time of insertion, or, more preferably, subsequent to insertion.

The following examples are offered by way of illustration, and not by way of limitation.

### EXAMPLES

### EXAMPLE 1

### COATING OF INTRA-ANATOMIC AORTIC GRAFTS WITH FIBRONECTIN.

The coating apparatus consisted of an overhead stirrer (Fisher Scientific) orientated horizontally. A conical stainless steel head was attached to the revolving chuck of the stirrer. One end of the intra-anatomic aortic graft was pulled up onto the conical head until held firmly. The other end was attached to a clip-swivel device that held the graft in a horizontal position, but allowed the graft to rotate along its axis. The stirrer was then set to rotate at 30 rpm so that the whole graft rotated along the horizontal axis at this speed. A 1% (w/w) fibronectin (Calbiochem, San Diego, CA) solution in sterile water was prepared. Two hundred microlitres of this solution was slowly pipetted as a 3 mm wide ring located 5 mm from the end of the graft fixed in the conical steel head over a period of 2 minutes as the graft rotated. The fibronectin was then dried under a stream of nitrogen as the graft continued to rotate. When dry, the graft was removed, turned around and the other end of the graft coated in the same manner. Using this method a flexible ring of fibronectin was deposited on both ends of the graft without compromise of the physical characteristics of the graft.

### EXAMPLE 2

### COATING OF INTRA-ANATOMIC AORTIC GRAFTS WITH POLY-L-LYSINE.

The coating apparatus consisted of a Fisher overhead stirrer orientated horizontally. A conical stainless steel head was attached to the revolving chuck of the stirrer. One end of the intra-anatomic aortic graft was pulled up onto the conical head until held firmly. The other end was attached to a clip-swivel device that held the graft in a horizontal position, but allowed the graft to rotate along its axis. The stirrer was set to rotate at 30 rpm so that the whole graft rotated along the horizontal axis at this speed. A 1% (w/w) poly-L-Lysine (Sigma, St. Louis, MO) solution in sterile water was prepared. Two hundred microlitres of this solution was slowly pipetted as a 3 mm wide ring located 5 mm from the end of the graft fixed in the conical steel head over a period of 2 minutes as the graft rotated. The poly-L-Lysine was then dried under a stream of nitrogen as the graft continued to rotate. When dry, the graft was removed, turned around and the other end of the graft coated in the same manner. Using this method a flexible ring of poly-L-Lysine was deposited on both ends of the graft without compromise of the physical characteristics of the graft.

### EXAMPLE 3

### COATING OF INTRA-ANATOMIC AORTIC GRAFTS WITH N-CARBOXYBUTYL CHITOSAN.

The coating apparatus consists of a Fisher overhead stirrer orientated horizontally. A conical stainless steel head is attached to the revolving chuck of the stirrer. One end of the intra-anatomic aortic graft is pulled up onto the conical head until held firmly. The other end is attached to a clip-swivel device that holds the graft in a horizontal position, but allows the graft to rotate along its axis. The stirrer is set to rotate at 30 rpm so that the whole graft rotates along the horizontal axis at this speed. A 1% (w/w) n-carboxybutyl chitosan (Carbomer, Westborough, MA) solution in sterile water is prepared. Two hundred microlitres of this solution is slowly pipetted as a 3 mm wide ring located 5 mm from the end of the graft fixed in the conical steel head over a period of 2 minutes as the graft rotates. The n-carboxybutyl chitosan is dried under a stream of nitrogen as the graft continues to rotate. When dry, the graft is removed, turned around and the other end coated in the same manner. Using this method a flexible ring of n-carboxybutyl chitosan is deposited on both ends of the graft without compromise of the physical characteristics of the graft.

### EXAMPLE 4

### COATING OF ANATOMIC AORTIC GRAFTS WITH BROMOCRIPTINE IN POLY(ETHYLENE VINYL ACETATE).

The coating apparatus consists of a Fisher overhead stirrer orientated horizontally. A conical stainless steel head is attached to the revolving chuck of the stirrer. One end of the intra-anatomic aortic graft is pulled up onto the conical head until held firmly. The other end is attached to a clip-swivel device that holds the graft in a horizontal position, but allows the graft to rotate along its axis. The stirrer is set to rotate at 30 rpm so that the whole graft rotates along the horizontal axis at this speed. A 4.5% w/w solution of EVA (60/40 ratio ethylene to vinyl acetate) (Polysciences USA) is prepared in dichloromethane. Bromocriptine mesylate (Sigma, St. Louis, MO) is dissolved/suspended in this solution at 5 mg/ml. Two hundred microlitres of this solution is slowly pipetted as a 3 mm wide ring located 5 mm from the end of the graft fixed in the conical steel head over a period of 2 minutes as the graft rotates. The EVA/bromocriptine is dried under a stream of nitrogen as the graft continues to rotate. When dry, the graft is removed, turned around and the other end of the graft coated in the same manner. Using this method a flexible ring of EVA/bromocriptine is deposited on both ends of the graft without compromise of the physical characteristics of the graft.

### EXAMPLE 5

### PREPARATION OF INFLAMMATORY MICROCRYSTALS (MONOSODIUM URATE MONOHYDRATE AND CALCIUM PYROPHOSPHATE DIHYDRATE).

Monosodium urate monohydrate (MSUM) microcrystals were grown. A solution of uric acid (certified A.C.S., Fisher Scientific) and sodium hydroxide at 55°C and pH 8.9 was left to stand overnight at room temperature. The crystals were rinsed several times with cold (4°C) distilled water and dried at 60°C for 12 hours in a circulating hot-air oven (Fisher, Isotemp).

Triclinic calcium pyrophosphate dihydrate (CPPD) crystals were prepared as follows. A 250 ml beaker containing 103 ml distilled water was heated in a water bath to 60 ± 2°C, and stirred constantly with a Teflon-coated stir bar. The stirring was slowed and 0.71 ml of concentrated hydrochloric acid and 0.32 ml of glacial acetic acid were added, followed by 0.6 g of calcium acetate (Fisher Certified Reagent). A 150 ml beaker containing 20 ml distilled water was heated to 60°C in the water bath, and 0.6 g calcium acetate added. The rate of stir was increased in the 250 ml beaker, and 2 g of calcium acid pyrophosphate added rapidly. When the CaH₂P₂O₇ had nearly all dissolved, the rate of stirring was reduced for 5 minutes, then over a period of 15 seconds, the contents of the small beaker were poured into the large beaker with vigorous stirring. In the preparation of subsequent batches, a minute amount of triclinic CPPD crystals was added to the large beaker as seed material. Stirring was discontinued, leaving a white gel. This was allowed to remain undisturbed in the cooling water bath. The pH of the supernatant was always less than 3.0. The gel collapsed as CPPD crystals formed in 24 hours. The crystals were washed in distilled water 3 times, washed in ethanol then acetone, and air dried.

### EXAMPLE 6

### COATING OF INTRA-ANATOMIC AORTIC GRAFTS WITH INFLAMMATORY MICROCRYSTALS (MONOSODIUM URATE MONOHYDRATE OR CALCIUM PYROPHOSPHATE DIHYDRATE).

The coating apparatus consists of a Fisher overhead stirrer orientated horizontally. A conical stainless steel head is attached to the revolving chuck of the stirrer. One end of the intra-anatomic aortic graft is pulled up onto the conical head until it is held firmly. The other end is attached to a clip-swivel device that holds the graft in a horizontal position, but allows the graft to rotate along its axis. The stirrer is set to rotate at 30 rpm so that the whole graft rotates along the horizontal axis at this speed. A 4.5% w/w solution of EVA (60/40 ratio ethylene to vinyl acetate) (Polysciences USA) is prepared in dichloromethane. Inflammatory microcrystals (MSUM or CPPD) are ground in a pestle and mortar to a particle size of 10 to 50 micrometers and suspended in the solution at 5 mg/ml. Two hundred microlitres of this suspension is slowly pipetted as a 3 mm wide ring located 5 mm from the end of the graft fixed in the conical steel head over a period of 2 minutes as the graft rotates. The EVA/microcrystals is then dried under a stream of nitrogen as the graft continues to rotate. When dry, the graft is removed, turned around and the other end of the graft coated in the same manner. Using this method a flexible ring of EVA/microcrystals is deposited on both ends of the graft without compromise of the physical characteristics of the graft.

### EXAMPLE 7

### COATING OF INTRA-ANATOMIC AORTIC GRAFTS WITH INFLAMMATORY MICROCRYSTALS (MONOSODIUM URATE MONOHYDRATE OR CALCIUM PYROPHOSPHATE DIHYDRATE).

A 1% w/w solution of Polyurethane (PU) (Medical grade, Thermomedics, Woburn, MA) is prepared in dichloromethane. Inflammatory microcrystals are ground in a pestle and mortar to a particle size of 10 to 50 micrometers and suspended in the solution at 2 mg/ml. Immediately prior to surgical insertion each end of the graft is inserted into the shaken suspension to a depth of approximately 5 mm for 2 seconds. The graft is air-dried (gently rotated by hand for 3 minutes). Using this method a flexible ring of EVA/microcrystals is deposited on both ends of the graft without compromise of the physical characteristics of the graft.

### EXAMPLE 8

### COATING OF INTRA-ANATOMIC AORTIC GRAFTS WITH BROMOCRIPTINE IN POLYURETHANE.

A 1% w/w solution of Polyurethane (PU) (Medical grade, Thermomedics, Woburn, MA) is prepared in dichloromethane. Bromocriptine mesylate (Sigma, St. Louis, MO) at 5% w/w to PU is dissolved/suspended in this solution. The solution is placed in a 5 ml Fisher TLC atomizer (Fisher Scientific). Prior to surgery the graft is suspended vertically in a fume hood and 1 ml of the solution sprayed (using nitrogen propellant) onto the bottom 1 cm of the graft by revolving the graft through 360 degrees. The graft is dried for 2 minutes and then the other end of the graft is sprayed in a similar manner. The graft is then further air dried (gently rotated by hand for 3 minutes). Using this method a flexible ring of bromocriptine/PU is deposited on both ends of the graft without compromise of the physical characteristics of the graft. It is envisaged that ultimately a bromocriptine/PU solution in DCM would be available to the surgeon in the form of a small aerosol can for the above procedure.

### EXAMPLE 9

### COATING OF INTRA-ANATOMIC AORTIC GRAFTS WITH INFLAMMATORY MICROCRYSTALS (MONOSODIUM URATE MONOHYDRATE OR CALCIUM PYROPHOSPHATE DIHYDRATE).

The coating apparatus consists of a Fisher overhead stirrer orientated horizontally. A conical stainless steel head is attached to the revolving chuck of the stirrer. One end of the intra-anatomic aortic graft is pulled up onto the conical head until it is held firmly. The other end is attached to a clip-swivel device that holds the graft in a horizontal position, but allows the graft to rotate along its axis. The stirrer is set to rotate at 30 rpm so that the whole graft rotates along the horizontal axis at this speed. A 4.5% w/w solution of Poly (lactide co-glycolide) (85:15) (IV 0.61) (Birmingham Polymers, Birmingham, AL) blended with methoxypolyethylene glycol 350 (MePEG 350) (Union Carbide, Danbury, CT) in a ratio of 80:20 w/w (PLGA:MePEG) is prepared in dichloromethane. Inflammatory microcrystals are suspended in the solution at 5 mg/ml. Two hundred microlitres of this suspension is slowly pipetted as a 3 mm wide ring located 5 mm from the end of the graft fixed in the conical steel head over a period of 2 minutes as the graft rotates. The PLGA/MePEG/inflammatory crystals are then dried under a stream of nitrogen as the graft continues to rotate. When dry, the graft is removed, turned around and the other end of the graft coated in the same manner. Using this method a flexible ring of PLGA/MePEG/microcrystals is deposited on both ends of the graft without compromise of the physical characteristics of the graft.

### EXAMPLE 10

### COATING OF INTRA-ANATOMIC AORTIC GRAFTS WITH SOLVENTS, SUCH AS ETHANOL OR CHLOROFORM.

A 1% w/w solution of Polyurethane (PU) (Medical grade, Thermomedics, Woburn, MA) is prepared in chloroform and stored until needed. Immediately prior to surgical insertion each end of the graft is dipped in the solution to a depth of approximately 5 mm for 2 seconds. The graft is immediately inserted into the animal before the polymer had fully dried. Using this method a flexible ring of PU containing significant amounts of chloroform is located at the required thrombogenic site without compromise of the physical characteristics of the graft. Alternatively, the PU can be dissolved at 1% (w/v) in a solution of chloroform: ethanol (80:20) to enable ethanol to be deposited at the site.

### EXAMPLE 11

### COATING OF INTRA-ANATOMIC AORTIC GRAFTS WITH ANGIOTENSIN 2 ENCAPSULATED IN POLYETHYLENE GLYCOL (PEG)

1.8 grams of Polyethylene glycol 1475 (Union Carbide, Danbury, CT) is placed in a flat-bottomed 20 ml glass scintillation vial and warmed to 50°C to melt the PEG in a water bath, 200 mg of glycerol (Fisher Scientific) is added. 2 mg of angiotensin 2 (Sigma, St. Louis, MO) is weighed into the vial and blended/dissolved into the melted PEG at 50°C. The vial is angled at 10 degrees in a water bath by use of a clamp. Each end of the graft is rotated in the molten formulation, so that a ring of material is deposited on the bottom 5 mm of the exterior surface of the graft. The graft is then cooled and stored at 4°C until use. Alternatively, to enable dipping immediately prior to surgery the PEG/angiotensin mixture is stored at 4°C until use. Immediately prior to surgery, the vial of PEG/angiotensin is warmed to 50°C for 2 minutes to melt and the graft is coated as described above.

### EXAMPLE 12

### COATING OF INTRA-ANATOMIC AORTIC GRAFTS WITH TRANSFORMING GROWTH FACTOR-β (TGF-β) IN CROSSLINKED HYALURONIC ACID.

The coating apparatus consists of a Fisher overhead stirrer orientated horizontally. A conical stainless steel head is attached to the revolving chuck of the stirrer. One end of the intra-anatomic aortic graft is pulled up onto the conical head until held firmly. The other end is attached to a clip-swivel device that holds the graft in a horizontal position, but allows the graft to rotate along its axis. The stirrer is set to rotate at 30 rpm so that the whole graft rotates along the horizontal axis at this speed. A 1% solution of hyaluronic acid (HA) (Sodium salt, Sigma, St. Louis, MO) in water, containing 30% glycerol (w/w to HA) (Fisher Scientific) and 8 mM 1-ethyl-3-(-3 dimethylaminopropyl) carbodiimide (EDAC) (Sigma, St. Louis, MO) is prepared by dissolution overnight. TGF-β (Calbiochem, San Diego, CA) is dissolved at 0.01 mg/ml in this solution. Two hundred microlitres of this solution is slowly pipetted as a 3 mm wide ring located 5 mm from the end of the graft fixed in the conical steel head over a period of 2 minutes as the graft rotates. The HA/glycerol/TGF-β solution is dried under a stream of nitrogen as the graft continues to rotate. When dry, the graft is removed, turned around and the other end coated in the same manner. Using this method a flexible ring of HA/glycerol/TGF-β is deposited on both ends of the graft without compromise of the physical characteristics of the graft.

### EXAMPLE 13

### COATING OF INTRA-ANATOMIC AORTIC GRAFTS WITH FIBROBLAST GROWTH FACTOR (FGF) IN CROSSLINKED CHITOSAN.

The coating apparatus consists of a Fisher overhead stirrer orientated horizontally. A conical stainless steel head is attached to the revolving chuck of the stirrer. One end of the intra-anatomic aortic graft is pulled up onto the conical head until held firmly. The other end is attached to a clip-swivel device that holds the graft in a horizontal position, but allows the graft to rotate along its axis. The stirrer is set to rotate at 30 rpm so that the whole graft rotates along the horizontal axis at this speed. A 1% solution of chitosan (Medical grade, Carbomer, Westborough, MA) in dilute acetic acid (pH 5), containing 30% glycerol (w/w to chitosan) (Fisher Scientific) and 0.5% glutaraldehyde (Sigma, St. Louis, MO) is prepared by dissolution overnight. FGF (Calbiochem, San Diego, CA) is dissolved at 0.01 mg/ml in this solution. Two hundred microlitres of this solution is slowly pipetted as a 3 mm wide ring located 5 mm from the end of the graft fixed in the conical steel head over a period of 2 minutes as the graft rotates. The chitosan/glycerol/FGF solution is dried under a stream of nitrogen as the graft continues to rotate. When dry, the graft is removed, turned around and the other end coated in the same manner. Using this method a flexible ring of chitosan/glycerol/FGF is deposited on both ends of the graft without compromise of the physical characteristics of the graft.

### EXAMPLE 14

### SCREENING PROCEDURE FOR ASSESSMENT OF PERIGRAFT REACTION

Large domestic rabbits are placed under general anesthetic. Using aseptic precautions, the infrarenal abdominal aorta is exposed and clamped at its superior and inferior aspects. A longitudinal arterial wall arteriotomy is performed and a 2 millimeter diameter, 1 centimeter long segment of PTFE graft is inserted within the aorta and the proximal and distal aspect of the graft is sewn so that the entire aortic blood flow is through the graft which is contained in the abdominal aorta in the manner of open surgical abdominal aortic repair in humans (except that no aneurysm is present in this model). The aortotomy is then surgically closed and the abdominal wound closed and the animal recovered.

The animals are randomized to receive standard PTFE grafts or grafts of which the middle 1 cm is coated alone circumferentially with nothing, or with an agent that induces a vessel wall reaction or adhesion between a stent graft and vessel wall alone or contained in a slow release, polymer such as polycaprolactone or polylactic acid.

The animals are sacrificed between 1 and 6 weeks post surgery, the aorta is removed en bloc and the area in relation to the graft is grossly examined for adhesive reaction. Any difference in morphology or histology of the vessel wall from portions of the artery which contain no graft, portion which contain graft without coating, and portion which contained graft with coating is noted.

### EXAMPLE 15

### ANIMAL ABDOMINAL AORTIC ANEURYSM MODEL

Pigs or sheep are placed under general anesthetic. Using aseptic precautions the abdominal aorta is exposed. The animal is heparinized and the aorta is cross clamped below the renal arteries and above the bifurcation. Collaterals are temporarily controlled with vessel loops or clips that are removed upon completion of the procedure. A longitudinal aortotomy is created in the arterial aspect of the aorta, and an elliptical shaped patch of rectus sheath from the same animal is sutured into the aortotomy to create an aneurysm. The aortic clamps from the lumbar arteries and collaterals are removed and the abdomen closed. After 30 days, the animal is reanesthesized and the abdominal wall again opened. A cutdown is performed on the iliac artery and through this, a stent graft is positioned across the infrarenal abdominal aorta aneurysm extending from normal infrarenal abdominal aorta above to normal infrarenal abdominal aorta below the surgically created aneurysm and the device is released in a conventional way.

Animals are randomized into groups of 5 receiving uncoated stent grafts, stent graft containing slow release polymer alone, and stent graft containing a biologically active or irritative substance as determined by the previously mentioned screening exam. After closure of the arteriotomy and of the abdominal wound, the animal is allowed to recover. At 6 weeks and 3 months post stent graft insertion, the animal is sacrificed and the aorta removed en bloc. The infrarenal abdominal aorta is examined for evidence of histologic reaction and perigraft leaking.

From the foregoing, it is appreciated that, although specific embodiments of the invention have been described herein for purposes of illustration, various modifications may be made without deviating from the scope of the invention. Accordingly, the invention is not limited except as by the appended claims.

## Claims

1. A stent graft, comprising an endoluminal stent and a graft, wherein said stent graft releases an agent which induces the *in vivo* adhesion of the stent graft to vessel walls.

2. A stent graft, comprising an endoluminal stent and a graft, wherein said stent graft induces or accelerates an *in vivo* fibrotic reaction causing said stent graft to adhere to vessel walls.

3. The stent graft according to claim 1 or 2 wherein said stent graft releases a vessel wall irritant.

4. The stent graft according to claim 3 wherein said vessel wall irritant is selected from the group consisting of talcum powder, metallic beryllium, and silica.

5. The stent graft according to claim I or 2 wherein said stent graft releases a component of extracellular matrix.

6. The stent graft according to claim 1 wherein said agent is fibronectin.

7. The stent graft according to claim 1 or 2 wherein said stent graft releases polylysine, or, ethylenevinylacetate.

8. The stent graft according to claim 1 or 2 wherein said stent graft releases an inflammatory cytokine selected from the group consisting of TGFβ, PDGF, VEGF, bFGF, TNFα, NGF, GM-CSF, IGF-a, IL-1, IL-8, IL-6, and growth hormone.

9. The stent graft according to claim 1 wherein said agent is an adhesive.

10. The stent graft according to claim 9 wherein said adhesive is cyanoacrylate.

11. The stent graft according to claim 1 or 2 wherein said stent graft is bifurcated.

12. The stent graft according to claim 1 or 2 wherein said stent graft is a tube graft.

13. The stent graft according to claim 12 wherein said stent graft is cylindrical.

14. The stent graft according to claim I or 2 wherein said stent graft is self-expandable.

15. The stent graft according to claim 1 or 2 wherein said stent graft is balloon-expandable.

16. The stent graft according to claim 1 or 2 wherein the distal ends of said stent graft are adapted to release an agent which induces adhesion.

17. The stent graft according to claim 1 or 2 wherein the entire body of said stent graft is adapted to release an agent that induces adhesion.

18. The stent graft according to claim 1 or 2, further comprising a coating which delays the onset of adhesion or fibrosis.

19. The stent graft according to claim 1 wherein said agent is first activated from a previously inactive agent to an active agent.

20. The stent graft according to claim 1 wherein said stent graft is activated from a previously inactive stent graft to a stent graft that induces or accelerates an *in vivo* fibrotic reaction.

21. A stent graft according to claim 1 or 2, for use in a method for treating patient having an aneurysm, comprising delivering to a patient said stent graft such that risk of rupture of the aneurysm is reduced.

22. The stent graft according to claim 21 wherein said aneurysm is an abdominal aortic aneurysm.

23. The stent graft according to claim 21 wherein said aneurysm is a thoracic aortic aneurysm.

24. The stent graft according to claim 21 wherein said aneurysm is an iliac aortic aneurysm.

25. A stent graft according to any one of claims 1 to 20 for use in a method for bypassing disease within a vessel, comprising delivering to a patient said stent graft, such that vessel contents bypass said diseased portion of said vessel.

26. A stent graft according to any one of claims 1 to 20 for use in a method for creating communication between an artery and a vein, comprising delivering to a patient, said stent graft such that a passageway is created between said artery and vein.

27. A stent graft according to any one of claims 1 to 20 for use in a method for creating communication between a first vein and a second vein, comprising delivering to a patient said stent graft, such that a passageway is created between said first and second veins.

28. The stent graft according to any one of claims 21, 25, 26 or 27 wherein said stent graft is delivered into a patient in a constrained form, and self-expands into place after release of a constraining device.

29. The stent graft according to any one of claims 21, 25, 26 or 27 wherein said stent graft is delivered to said patient by balloon catheter.

30. A method of manufacturing an adhesive stent graft, comprising coating a stent graft with an agent which induces adhesion of the stent graft to vessel walls.

31. The method according to claim 30 wherein said stent graft is coated by spraying, dipping, or wrapping said stent graft with said agent.

32. The method according to claim 30 wherein said agent further comprises a polymer.

33. The method according to claim 30 wherein said agent is a vessel wall irritant.

34. The method according to claim 30 wherein said agent is an inflammatory cytokine.

35. The method according to claim 30 wherein said agent is an inflammatory crystal.

36. The method according to claim 30 wherein said agent is bFGF.

## Patentansprüche

1. Stent-Transplantat, das einen endoluminalen Stent und ein Transplantat umfaßt, wobei besagtes Stent-Transplantat ein Agens freisetzt, das die *in-vivo*-Adhäsion des Stent-Transplantats an Gefäßwänden induziert.

2. Stent-Transplantat, das einen endoluminalen Stent und ein Transplantat umfaßt, **dadurch gekennzeichnet, daß** besagtes Stent-Transplantat eine fibrotische *in-vivo*-Reaktion induziert oder beschleunigt, die bewirkt, daß besagtes Stent-Transplantat an Gefäßwänden anhaftet.

3. Stent-Transplantat nach Anspruch 1 oder 2, **dadurch, gekennzeichnet, daß** besagtes Stent-Transplantat ein Gefäßwand-Reizmittel freisetzt.

4. Stent-Transplantat nach Anspruch 3, **dadurch gekennzeichnet, daß** besagtes Gefäßwand-Reizmittel aus der Gruppe ausgewählt ist, die aus Talkpulver, metallischem Beryllium und Siliciumdioxid besteht.

5. Stent-Transplantat nach Anspruch 1 oder 2, **dadurch gekennzeichnet, daß** besagtes Stent-Transplantat eine Komponente von extrazellulärer Matrix freisetzt.

6. Stent-Transplantat nach Anspruch 1, **dadurch gekennzeichnet, daß** besagtes Agens Fibronectin ist.

7. Stent-Transplantat nach Anspruch 1 oder 2, **dadurch gekennzeichnet, daß** besagtes Stent-Transplantat Polylysin oder Ethylenvinylacetat freisetzt.

8. Stent-Transplantat nach Anspruch 1 oder 2, **dadurch gekennzeichnet, daß** besagtes Stent-Transplantat ein Entzündungscytokin freisetzt, das ausgewählt ist aus der Gruppe, die aus TGFβ, PDGF, VEGF, bFGF, TNFα, NGF, GM-CSF, IGF-a, IL-1, IL-8, IL-6 und Wachstumshormon besteht.

9. Stent-Transplantat nach Anspruch 1, **dadurch gekennzeichnet, daß** besagtes Agens ein Klebstoff ist.

10. Stent-Transplantat nach Anspruch 9, **dadurch gekennzeichnet, daß** besagter Klebstoff Cyanacrylat ist.

11. Stent-Transplantat nach Anspruch 1 oder 2, **dadurch gekennzeichnet, daß** besagtes Stent-Transplantat gegabelt ist.

12. Stent-Transplantat nach Anspruch 1 oder 2, **dadurch gekennzeichnet, daß** besagtes Stent-Transplantat ein Rundstiellappen-Transplantat ist.

13. Stent-Transplantat nach Anspruch 12, **dadurch gekennzeichnet, daß** besagtes Stent-Transplantat zylindrisch ist.

14. Stent-Transplantat nach Anspruch 1 oder 2, **dadurch gekennzeichnet, daß** besagtes Stent-Transplantat selbst-expandierbar ist.

15. Stent-Transplantat nach Anspruch 1 oder 2, **dadurch gekennzeichnet, daß** besagtes Stent-Transplantat ballonexpandierbar ist.

16. Stent-Transplantat nach Anspruch 1 oder 2, **dadurch gekennzeichnet, daß** die distalen Enden besagten Stent-Transplantats angepaßt sind, um ein Agens freizusetzen, das Adhäsion induziert.

17. Stent-Transplantat nach Anspruch 1 oder 2, **dadurch gekennzeichnet, daß** der gesamte Körper besagten Stent-Transplantats angepaßt ist, um ein Agens freizusetzen, das Adhäsion induziert.

18. Stent-Transplantat nach Anspruch 1 oder 2, **dadurch gekennzeichnet, daß** es weiter eine Beschichtung umfaßt, die das Einsetzen der Adhäsion oder Fibröse verzögert.

19. Stent-Transplantat nach Anspruch 1, **dadurch gekennzeichnet, daß** besagtes Agens zunächst aus einem zuvor inaktiven Agens zu einem aktiven Agens aktiviert wird.

20. Stent-Transplantat nach Anspruch 1, **dadurch gekennzeichnet, daß** besagtes Stent-Transplantat aus einem zuvor inaktiven Stent-Transplantat zu einem Stent-Transplantat, das eine fibrotische *in-vivo*-Reaktion induziert oder beschleunigt, aktiviert wird.

21. Stent-Transplantat nach Anspruch 1 oder 2, zur Verwendung in einem Verfahren zur Behandlung eines Patienten mit einem Aneurysma, welches umfaßt, daß einem Patienten besagtes Stent-Transplantat so zugeführt wird, daß das Risiko einer Ruptur des Aneurysmas verringert wird.

22. Stent-Transplantat nach Anspruch 21, **dadurch gekennzeichnet, daß** besagtes Aneurysma ein Bauchaorta-Aneurysma ist.

23. Stent-Transplantat nach Anspruch 21, **dadurch gekennzeichnet, daß** besagtes Aneurysma Brustaorta-Aneurysma ist.

24. Stent-Transplantat nach Anspruch 21, **dadurch gekennzeichnet, daß** besagtes Aneurysma ein Darmbeinaorta-Aneurysma ist.

25. Stent-Transplantat nach einem der Ansprüche 1 bis 20, zur Verwendung in einem Verfahren zum Überbrücken einer Erkrankung in einem Gefäß, welches umfaßt, daß einem Patienten besagtes Stent-Transplantat so zugeführt wird, daß Gefäßinhalte besagten krankheitsbefallenen Abschnitt besagten Gefäßes umgehen.

26. Stent-Transplantat nach einem der Ansprüche 1 bis 20, zur Verwendung in einem Verfahren zum Schaffen einer Verbindung zwischen einer Arterie und einer Vene, welches umfaßt, daß einem Patienten besagtes Stent-Transplantat so zugeführt wird, daß ein Durchlaß zwischen besagter Arterie und Vene geschaffen wird.

27. Stent-Transplantat nach einem der Ansprüche 1 bis 20, zur Verwendung in einem Verfahren zum Schaffen einer Verbindung zwischen einer ersten Vene und einer zweiten Vene, welches umfaßt, daß einem Patienten besagtes Stent-Transplantat so zugeführt wird, daß ein Durchlaß zwischen besagter ersten und besagter zweiten Vene geschaffen wird.

28. Stent-Transplantat nach einem der Ansprüche 21, 25, 26 oder 27, **dadurch gekennzeichnet, daß** besagtes Stent-Transplantat einem Patienten in einer zusammengezogenen Form zugeführt wird und sich nach Lösen einer zusammenziehenden Einheit an Ort und Stelle von selbst expandiert.

29. Stent-Transplantat nach einem der Ansprüche 21, 25, 26 oder 27, **dadurch gekennzeichnet, daß** besagtes Stent-Transplantat besagtem Patienten durch Ballonkatheter zugeführt wird.

30. Verfahren zur Herstellung eines adhäsiven Stent-Transplantats, welches umfaßt, daß ein Stent-Transplantat mit einem Agens beschichtet wird, das Adhäsion des Stent-Transplantats an Gefäßwänden induziert.

31. Verfahren nach Anspruch 30, **dadurch gekennzeichnet, daß** besagtes Stent-Transplantat durch besprühen, tauchen oder umwickeln besagten Stent-Transplantats mit besagtem Agens beschichtet wird.

32. Verfahren nach Anspruch 30, **dadurch gekennzeichnet, daß** besagtes Agens weiter ein Polymer umfaßt.

33. Verfahren nach Anspruch 30, **dadurch gekennzeichnet, daß** besagtes Agens ein Gefäßwand-Reizmittel ist.

34. Verfahren nach Anspruch 30, **dadurch gekennzeichnet, daß** besagtes Agens ein Entzündungscytokin ist.

35. Verfahren nach Anspruch 30, **dadurch gekennzeichnet, daß** besagtes Agens ein Entzündungskristall ist.

36. Verfahren nach Anspituch 30, **dadurch gekennzeichnet, daß** besagtes Agens bFGF ist.

## Revendications

1. Greffon extenseur, comprenant un extenseur endoluminal et un greffon, ledit greffon extenseur libérant un agent qui induit l'adhérence *in vivo* du greffon extenseur aux parois d'un vaisseau.

2. Greffon extenseur, comprenant un extenseur endoluminal et un greffon, ledit greffon extenseur induisant ou accélérant une réaction fibrotique *in vivo* provoquant l'adhérence dudit greffon extenseur aux parois d'un vaisseau.

3. Greffon extenseur suivant la revendication 1 ou 2, ledit greffon extenseur libérant un agent irritant pour les parois d'un vaisseau.

4. Greffon extenseur suivant la revendication 3, dans lequel ledit agent irritant pour les parois d'un vaisseau est choisi dans le groupe consistant en la poudre de talc, le béryllium métallique et la silice.

5. Greffon extenseur suivant la revendication 1 ou 2, ledit greffon extenseur libérant un constituant de la matrice extracellulaire.

6. Greffon extenseur suivant la revendication 1, dans lequel ledit agent est la fibronectine.

7. Greffon extenseur suivant la revendication 1 ou 2, ledit greffon extenseur libérant de la polylysine ou de l'éthylène-acétate de vinyle.

8. Greffon extenseur suivant la revendication 1 ou 2, ledit greffon extenseur libérant une cytokine inflammatoire choisie dans le groupe consistant en TGFβ, PDGF, VEGF, bFGF, TNFα, NGF, GM-CSF, IGF-a, IL-1, IL-8, IL-6 et l'hormone de croissance.

9. Greffon extenseur suivant la revendication 1, dans lequel ledit agent est un adhésif.

10. Greffon extenseur suivant la revendication 9, dans lequel ledit adhésif est un cyanoacrylate.

11. Greffon extenseur suivant la revendication 1 ou 2, ledit greffon extenseur étant bifurqué.

12. Greffon extenseur suivant la revendication 1 ou 2, ledit greffon extenseur étant un greffon tubulaire.

13. Greffon extenseur suivant la revendication 12, ledit greffon extenseur étant cylindrique.

14. Greffon extenseur suivant la revendication 1 ou 2, ledit greffon extenseur étant apte à l'auto-expansion.

15. Greffon extenseur suivant la revendication 1 ou 2, ledit greffon extenseur étant apte à l'expansion par ballonnets.

16. Greffon extenseur suivant la revendication 1 ou 2, dans lequel les extrémités distales dudit greffon extenseur sont adaptées à la libération d'un agent qui induit une adhérence.

17. Greffon extenseur suivant la revendication 1 ou 2, dans lequel le corps total dudit greffon extenseur est adapté à la libération d'un agent qui induit une adhérence.

18. Greffon extenseur suivant la revendication 1 ou 2, comprenant en outre un revêtement qui retarde le début d'adhérence ou de fibrose.

19. Greffon extenseur suivant la revendication 1, dans lequel ledit agent subit tout d'abord une activation d'un agent auparavant inactif en un agent actif.

20. Greffon extenseur suivant la revendication 1, ledit greffon extenseur étant activé d'un greffon extenseur auparavant inactif en un greffon extenseur qui induit ou accélère une réaction fibrotique in vivo.

21. Greffon extenseur suivant la revendication 1 ou 2, destiné à être utilisé dans une méthode de traitement d'un patient souffrant d'un anévrisme, comprenant l'administration à un patient dudit greffon extenseur de telle sorte que le risque de rupture de l'anévrisme soit réduit.

22. Greffon extenseur suivant la revendication 21, dans lequel ledit anévrisme est un anévrisme aortique abdominal.

23. Greffon extenseur suivant la revendication 21, dans lequel ledit anévrisme est un anévrisme aortique thoracique.

24. Greffon extenseur suivant la revendication 21, dans lequel ledit anévrisme est un anévrisme aortique iliaque.

25. Greffon extenseur suivant l'une quelconque des revendications 1 à 20, destiné à être utilisé dans une méthode pour éviter une partie malade dans un vaisseau, comprenant l'administration à un patient dudit greffon extenseur de telle sorte que le contenu du vaisseau évite ladite partie malade dudit vaisseau.

26. Greffon extenseur suivant l'une quelconque des revendications 1 à 20, destiné à être utilisé dans une méthode pour créer une communication entre une artère et une veine, comprenant l'administration à un patient dudit greffon extenseur de telle sorte qu'une voie de passage soit créée entre ladite artère et ladite veine.

27. Greffon extenseur suivant l'une quelconque des revendications 1 à 20, destiné à être utilisé dans une méthode pour créer une communication entre une première veine et une seconde veine, comprenant l'administration à un patient dudit greffon extenseur de telle sorte qu'une voie de passage soit créée entre lesdites première et seconde veines.

28. Greffon extenseur suivant l'une quelconque des revendications 21, 25, 26 et 27, qui est délivré sous forme entravée dans le corps d'un patient et qui subit une auto-expansion en place après libération d'un dispositif de contention.

29. Greffon extenseur suivant l'une quelconque des revendications 21, 25, 26 et 27, ledit greffon extenseur étant délivré audit patient par un cathéter à ballonnet.

30. Procédé pour la production d'un greffon extenseur adhésif, comprenant le revêtement d'un greffon extenseur avec un agent qui induit une adhérence du greffon extenseur aux parois d'un vaisseau.

31. Procédé suivant la revendication 30, dans lequel ledit greffon extenseur est revêtu en pulvérisant ledit agent, en immergeant ledit greffon extenseur ou en entourant ledit greffon avec ledit agent.

32. Procédé suivant la revendication 30, dans lequel ledit agent comprend en outre un polymère.

33. Procédé suivant la revendication 30, dans lequel ledit agent est un agent irritant pour les parois d'un vaisseau.

34. Procédé suivant la revendication 30, dans lequel ledit agent est une cytokine inflammatoire.

35. Procédé suivant la revendication 30, dans lequel ledit agent est un cristal inflammatoire.

36. Procédé suivant la revendication 30, dans lequel ledit agent consiste en bFGF.
